# EUROPEAN PATENT SPECIFICATION

(11) **EP 2 250 186 B1**
(45) Date of publication and mention of the grant of the patent: **13.08.2014**
(21) Application number: 09708190.5
(22) Date of filing: 05.01.2009
(51) Int. Cl.: C07K 1/12, G01N 33/68

(54) **HIGH PRESSURE ENZYMATIC DIGESTION SYSTEM FOR PROTEIN CHARACTERIZATION**
ENZYMATISCHES HOCHDRUCKVERDAUUNGSSYSTEM ZUR PROTEINCHARAKTERISIERUNG
SYSTÈME DE DIGESTION ENZYMATIQUE HAUTE PRESSION POUR CARACTÉRISATION DE PROTÉINES

(30) Priority: 07.02.2008 US 26845 P; 31.07.2008 US 183219
(43) Date of publication of application: 17.11.2010
(73) Proprietor: Battelle Memorial Institute, Richland, WA 99352 (US)
(72) Inventor: LOPEZ-FERRER, Daniel, Richland, WA 99354 (US)
(74) Representative: Bradley, Adrian
(86) International application number: PCT/US2009/030135
(87) International publication number: WO 2009/099685

(56) References cited:
- ANONYMOUS: "Independent research group reports that pressure BioSciences' award winning Proteo-Solve-LRS technology played a significant role in the identification of potential biomarkers in breast and colon cancer tissue" PR NEWSWIRE, [Online] 13 December 2007 (2007-12-13), XP002538773 Retrieved from the Internet: URL:http://www.prnewswire.com/cgi-bin/stor ies.pl?ACCT=109&STORY=/www/story/12-13-200 7/0004722216&EDATE=> [retrieved on 2009-07-27]
- LOPEZ-FERRER D ET AL: "Sample treatment for protein identification by mass spectrometry-based techniques" TRENDS IN ANALYTICAL CHEMISTRY, vol. 25, no. 10, November 2006 (2006-11), pages 996-1005, XP025029883 ISSN: 0165-9936
- GARRETT P E ET AL: "Tired of the same old grind in the new genomics and proteomics era?" TARGETS, ELSEVIER, vol. 1, no. 5, 1 November 2002 (2002-11-01), pages 156-162, XP004886618 ISSN: 1477-3627
- CHICON ROSA ET AL: "Changes in chymotrypsin hydrolysis of beta-lactoglobulin A induced by high hydrostatic pressure" JOURNAL OF AGRICULTURAL AND FOOD CHEMISTRY, vol. 54, no. 6, March 2006 (2006-03), pages 2333-2341, XP002538774 ISSN: 0021-8561
- LÓPEZ-FERRER DANIEL ET AL: "Application of pressurized solvents for ultrafast trypsin hydrolysis in proteomics: proteomics on the fly." JOURNAL OF PROTEOME RESEARCH AUG 2008, vol. 7, no. 8, 7 August 2008 (2008-08-07), pages 3276-3281, XP002538775 ISSN: 1535-3893
- LOPEZ-FERRER D ET AL: "Ultra fast trypsin digestion of proteins by high intensity focused ultrasound", JOURNAL OF PROTEOME RESEARCH, vol. 4, no. 5, September 2005 (2005-09), pages 1569-1574, ISSN: 1535-3893

## Description

The present invention generally refers to analytical methods and, more particularly, to the large scale analysis of proteins or proteomics.

Modern scientific methods in biology have led to a variety of opening technologies, such as genomics, proteomics, metabolomics, which have been utilized to understand relationships and interactions in biological systems. These methods and sciences have also contributed greatly to the advancement of clinical and biotechnological analyses. One of the problems that exists in these disciplines is the time required to prepare and process samples. While various advancements have been made in the reduction of analysis time, one of the key bottlenecks in this process occurs during the sample processing and preparation period. This is particularly true when large scale studies need to be done and consequently a large amount of samples need to be processed. While various schemes have been utilized to attempt to increase the throughput of samples by speeding up the sample preparation process, none of these have been adopted with universal appeal.

In proteomics, the typical sample preparation step, includes the digestion of a complex protein sample, by being incubated with an enzyme, in a buffered medium for a defined period of time, typically overnight or around 12 hours. This extended time requirement slows down the through processing of protein samples and makes protein digestion one of the most time-consuming steps in proteoanalysis workflow. In addition, because such a preparation process is generally carried out manually, associated risk related to operator's error can also negatively impact the analysis. Additionally, manual sample processing can give rise to larger sample/reagent consumption and increased costs due to the labor involved. When working with very small sample sizes which is often the case for clinical applications, automated and quick protein characterization is imperative to limit contamination and other operator-related sources of error, and to bring the use of LC-MS analysis to the next level of efficiency and productivity.

The document "Sample treatment for protein identification by mass spectrometry-based techniques" Trends in Analytical Chemistry 25, no. 10. 996-1005, November 2006 to D. López -Ferrer et al., discloses an overview of methods for sample treatment for protein identification by mass spectrometry and discuss enzymatic digestion of proteins using microwaves, ultrasound and combinations thereof.

The document "Tired of the same old grind in the new genomics and proteomics era?", Targets 1, no 5, 156-162, 1 November 2002 to Garrett P.E. et al., discloses pressure cycling technology for use in cell lysis in which it is desirable to obtain an intact protein.

The document "Ultra fast trypsin digestion of proteins by high intensity focused ultrasound", Journal of Proteome Research, 2005, 4, 1569-1574 to López -Ferrer. D et al., discloses proteolytic digestion of proteins under an ultrasound field.

The document "Changes in chymotrypsin hydrolysis of beta-lactoglobulin A induced by high hydrostatic pressure", Journal of Agricultural and Food Chemistry, vol. 54, no. 6, March 2006, pages 2333-2341 by Rosa Chicón et al, discloses chymotrypsin-mediated proteolysis of beta-lactoglobulin subjected to high pressures up to 400 MPa.

Therefore, what is needed is a method for increasing the rate at which materials such as proteins can be prepared for analysis and analyzed. What is also needed is a method which prepares these materials for analysis which does not negatively impact the analytical workflow utilized therewith. The present invention meets these needs.

Additional advantages and novel features of the present invention will be set forth as follows and will be readily apparent from the descriptions and demonstrations set forth herein. Accordingly, the following descriptions of the present inventions should be seen as illustrative of the invention and not as limiting in any way. Various advantages and novel features of the present invention are described herein and will be further made apparent to those skilled in the art from the following detailed description.

### SUMMARY

The present invention is a method according to claim 1 for obtaining samples for proteomic analysis that utilizes pressure and a trypsin to obtain a processing sample in a significantly shorter period of time than prior art methods and which maintains the integrity of the processing sample through the preparatory process. In the present invention, a sample is subjected to a preselected pressure typically some where between 0.5 psi and 100kpsi, for selected periods or intervals of time typically between 5 and 1800 seconds. Through this pressurization process various other agents such as chemicals, enzymes, microwaves, sound, ultrasound, heat, light, and combinations thereof may also be combined with the pressure to affect a desired result and produce a sample having desired characteristics.

In one embodiment of the invention, a sample and a trypsin are combined and subjected to a pressure, preferably a pressure cycle range that varies between 0 to 35 kpsi, for a period of time of preferably less than 60 seconds. This process results in producing a sample suitable for analysis.

This method can be embodied in a system for proteomic analysis which includes a sample preparation device that treats a protein sample with pressure and a trypsin, examples of which have been discussed earlier. This sample preparation device is then operatively connected to an analytical instrument, which allows for transfer of the treated sample to the analytical instrument for analysis to take place. In one embodiment of the invention the analytical instrument is a high pressure liquid chromatography (LC) system with a pressurized sample loop. This device may then be coupled to another analytical instrument such as a mass spectrometry instrument, or other device. Various modifications and alterations may be made to the system to perform other tasks such as tagging a process sample with a material such as a radioisotope.

While these examples have been provided, it is to be distinctly understood that the invention is not limited thereto.

Various advantages and novel features of the present invention are described herein and will become further readily apparent to those skilled in this art from the following detailed description. In the preceding and following descriptions the preferred embodiment of the invention, by way of illustration of a best mode contemplated for carrying out the invention have been provided. The drawings and description of the preferred embodiment set forth hereafter are to be regarded as illustrative, and not as restrictive.

### BRIEF DESCRIPTION OF THE DRAWINGS

Figures 1a and 1b show the results of testing of one embodiment of the present invention.
Figure 2a and 2B show additional results of testing of one embodiment of the present invention.
Figure 3a-3f shows comparative results of one embodiment of the present invention.
Figure 4a-4c shows additions comparative results of an embodiment of the present invention.
Figure 5a-5c shows various embodiments of an apparatus suitable for working the present invention
Figure 6a-6g shows one embodiment of an apparatus suitable for working the present invention.
Figure 7a-7c shows various results of one embodiment of the present invention.

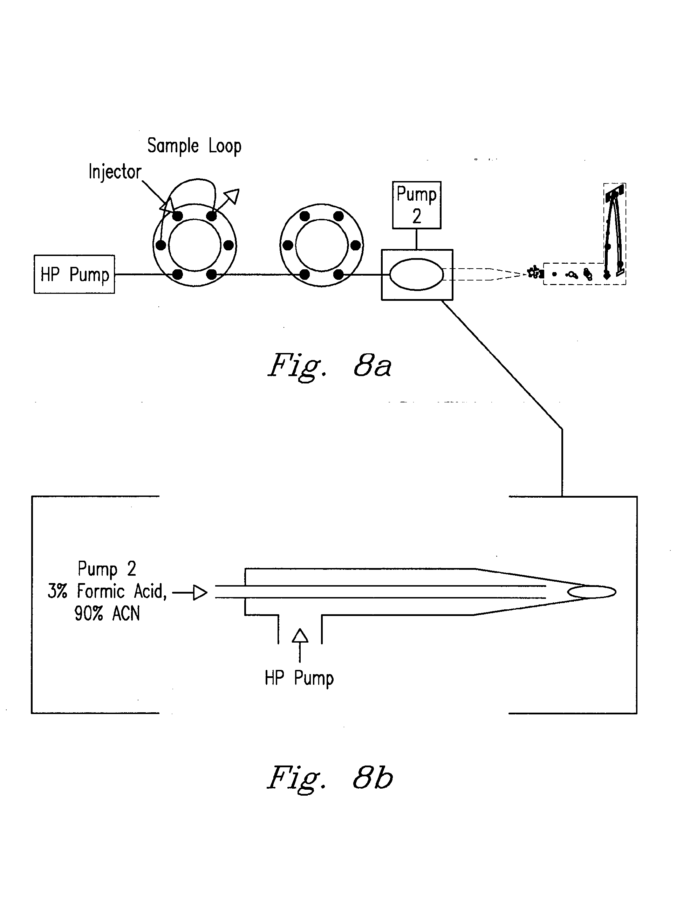

Figure 8a-8b shows one embodiment of an apparatus suitable for working the present invention.
Figure 9a-9c shows various results of one embodiment of the present invention.
Figure 10a-10b shows results of one embodiment of the present invention.
Figure 11a-11d shows various results of one embodiment of the present invention.
Figure 12 shows another embodiment of an apparatus suitable for working the present invention.

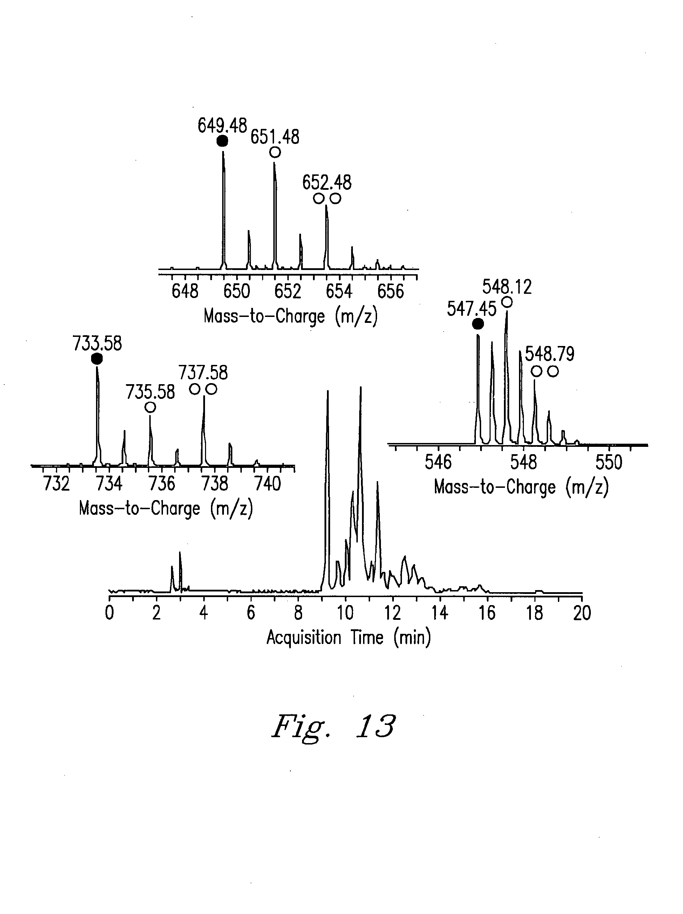

Figure 13 shows the results of testing performed with the embodiment shown in Fig. 12.

### DETAILED DESCRIPTION OF THE INVENTION

In one embodiment, the present invention is a new method for rapid proteolytic digestion of proteins under high pressure that uses pressure cycling technology in the range of 5 to 35 kpsi w to prepare samples for proteomic analysis. While these specific examples are shown it is to be distinctly understood that the invention is not limited thereto but maybe variously alternatively embodied according to the needs and necessities of a particular user. In the method of the present invention successful in-solution digestions of single proteins and complex protein mixtures were achieve in 60 s utilizing this method and then analyzed by reversed phase liquid chromatography-electrospray ionization ion trap-mass spectrometry. The results of the samples prepared by this method coordinated with the results of samples prepared by the traditional prior art method. However, the method described in the present invention provides greatly simplified sample processing, easy implementation, no cross contamination among samples, and cost effectiveness.

In one set of experiments described hereafter one embodiment of the present invention was compared to a common overnight digestion process. In this particular application, bovine serum albumin (BSA) was used as a standard protein to evaluate the method under different conditions. First, 6 mg of BSA was denatured in 8 M urea and reduced with 10 mM DTT in 25 mM ammonium bicarbonate (pH 8.25) at 37 °C for 1 h. Iodoacetamide was added to a final concentration of 50 mM, and the resulting mixture was incubated at room temperature in the dark for 45 min. Twelve 50- 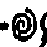 g aliquots were diluted 4 fold to reduce the urea concentration, using either 25 mM ammonium bicarbonate, 20% MeOH, or 80% MeOH. Trypsin was added (1:50 protease-to-protein ratio), to a final volume of 1.4 mL and the solutions were placed in pulse tubes. The Barocycler™ NEP-3229 instrument and disposable polypropylene PULSE tubes FT-500 were obtained from Pressure BioSciences (West Bridgewater, MA, USA) and were used for all experiments. The pulse tubes were subjected to the Barocycler™ program, using 4 or 8 pressure pulses for a total of 1 minute per run. Finally, the enzymatic digests were transferred to new centrifuge tubes, acidified, and frozen with liquid N₂ to stop the reaction. The samples were then dried down by centrifugal evaporation and stored at -20 ºC.

The *Shewanella oneidensis,* strain MR-1, whole cell protein tryptic digest was prepared by lysing by bead beating, using 0.1 mm zirconia/silica beads in a mini-bead beater for 180 s at 4500 rpm. The lysate was collected and placed immediately on ice to inhibit proteolysis, then denatured with 8 M urea, 25 mM ammonium bicarbonate, 10 mM DTT, (pH 8), and incubated for 1 h at 37 °C. Iodoacetamide was added to a final concentration of 50 mM, and the resultant mixture was incubated for 45 min at room temperature in the dark. The mixture was diluted 4 fold, and following the addition of trypsin (1:50 protease-to-protein ratio), was incubated either overnight at 37 °C overnight or for 1 min using PCT at 35 kpsi.

A solution with a final concentration of 1 µM protein in 12.5 mM ammonium bicarbonate was prepared for the myoglobin experiments. Trypsin was added and the samples were digested (1:50 protease-to-protein ratio) during the pressure cycles in the Barocycler™. After treatment, 500 fmol of the protein digest was analyzed by LC-MS/MS. Separations were performed using a 40-nL enrichment column and 43 mm x 75 µm analytical column packed with 5 µm ZORBAX 300SB C18 particles. A flow rate of 1 µL/min was employed for enrichment and 600 nL/min afterwards. Peptides were eluted using a 5 min gradient from 5% to 90% Solvent B (0.5% formic acid, 90% acetonitrile; Solvent A: 0.5% formic acid in water: acetonitrile 97:3), with a separation window of ∼2 min. The total analysis time was 12 min. Each sample was analyzed in triplicate. To prevent cross contamination among different samples, a blank was run between each set of replicates.

The data were acquired in survey scans from 500 to 1600 amu (3 microscans) followed by five data dependent MS/MS scans, using an isolation width of 3 amu, a normalized collision energy of 35%, and a dynamic exclusion period of 2 min. MS/MS data were analyzed using Spectrum Mill software against an in-house FASTA database that contained *S. oneidensis* MR-1 and BSA proteins. Spectra that matched to BSA were manually verified.

For the complex protein mixture analysis, 2 µg of the S. *oneidensis* digest were analyzed using a custom-built capillary LC system coupled online to a linear ion trap mass spectrometer with an in-house developed ESI source. The LTQ mass spectrometer was operated in a data-dependent MS/MS mode (m/z 400-2,000), in which a full MS scan was followed by ten MS/MS scans, using a normalized collision energy of 35% with a dynamic exclusion of 1 min. Protein identification was carried out using SEQUEST to deduce protein sequences from the S. *oneidensis* MR-1 genome sequence. Database search parameters included a dynamic modification search (i.e., the presence and absence of the modification was searched) for Met oxidation and a static search (i.e., presence of the modification was searched only) for carbamidomethylation on Cys. Error rates for peptide identifications were calculated as reported previously.

To study myoglobin folding, the protein was directly infused by a syringe pump at 1 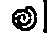 L/min either with or without previous pressure treatment, into an Agilent TOF MS through an ESI interface. MS data were recorded over an m/z range of 500-2500 at a scan rate of 1 scan/sec.

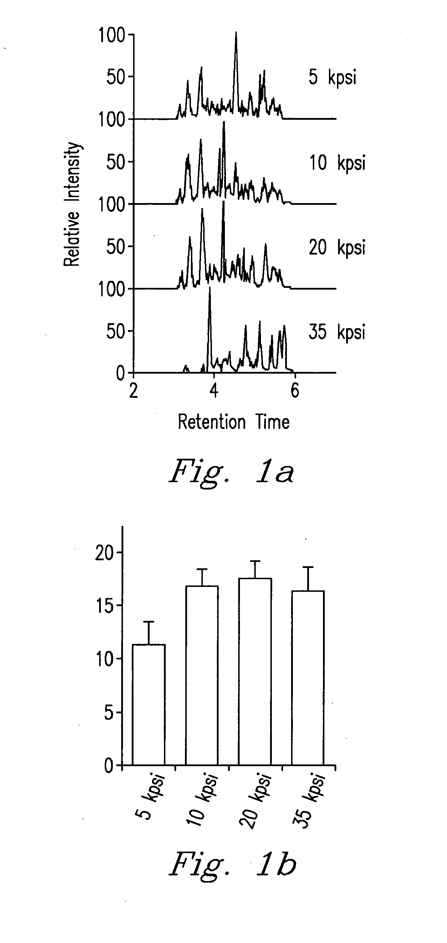

Referring now to Figures 1a, and 1b, the effect of enzyme activity in terms of protein proteolytic products at 5,10, 20, and 35 kpsi for 60 s at each pressure is shown. The chromatograms in Figure 1a indicate that trypsin activity was not compromised at any of the pressures. However, as is shown in Figure 1b, digestion of the identified peptides was not as complete at 5 kpsi as those achieved at higher pressures, even though the chromatograms at 5,10, and 20 kpsi are similar. Although chromatograms belonging to the 35 kpsi samples look significantly different as compare with the others. Manual inspection of the MS spectra showed the same peptides between chromatograms. When pressure was applied to solutions that contained BSA in the absence of trypsin, no protein degradation products were observed, which indicates that the pressure treatment itself did not cause protein fragmentation.

To evaluate the influence of rapid cycling between high and low pressures on trypsin activity, BSA was digested under pressure, using either 4 or 8 differential pressure cycles for a total of 60s. To further analyze the combined effect of pressure in the presence of an organic solvent for a trypsin digestion, identical BSA protein aliquots were subjected to pressure-digestion at 35 kpsi in the presence of 1) ammonium bicarbonate, 2) an 80:20 (v/v) mixture of ammonium bicarbonate:methanol, and 3) a 20:80 (v/v) mixture of ammonium bicarbonate: methanol. The properties of enzymes in mixed organic-aqueous solvent systems are influenced by factors such as protein structure, presence of phase interfaces, dielectric constants, etc.; all of which contribute to the performance of an enzyme in its biocatalytic system.

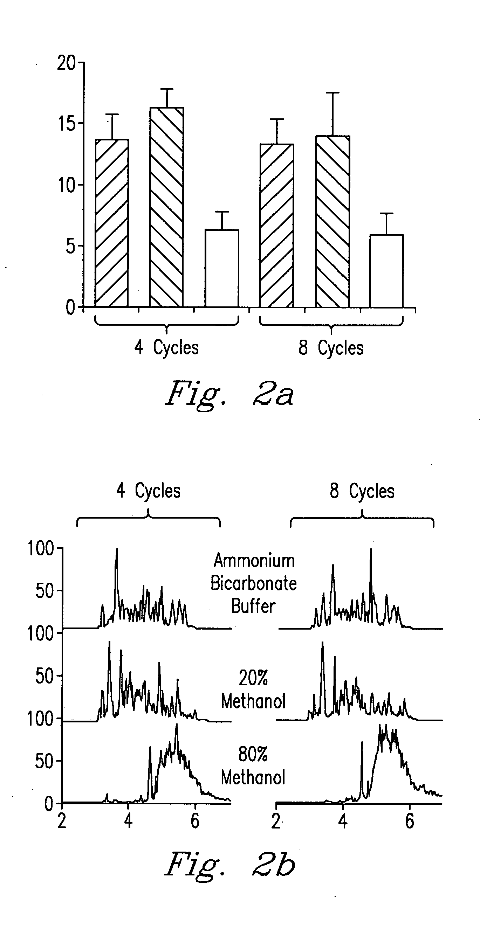

The histogram in Figure 2a shows that nearly identical results in terms of the number of unique peptide identifications were obtained for samples digested in comparable digestion buffers, regardless of the number of cycles. The chromatographic profiles in Figure 2b are also very similar for comparable buffers. A comparison of these results obtained at cyclic pressures to those obtained at constant pressure revealed no significant differences, which suggests that at least for trypsin, there is no significant effect in activity due to the number of differential pressure cycles used with PCT during digestion. In addition, the number of identified peptides was not significantly different between aqueous (i.e., ammonium bicarbonate) and 20% methanol digestions for either the 4- and 8-cycle protocols (Figure 2a).

This embodiment of the present invention was also evaluated by applying PCT to a proteomics sample and then analyzing it using a shotgun proteomics approach. A total proteome extract from a preparation of S. *oneidensis* cells was separated into two identical aliquots, one of which was subjected to a PCT-assisted digestion at 35 kpsi for 8 cycles during a 60 s time frame and the other, to a trypsin digestion following the conventional overnight approach for comparative purposes. PCT conditions reflected the highest number of cycles and the highest pressure that the Barocycler™ is capable of operating that was shown previously to achieve a good trypsin activity. The digested peptide mixtures were analyzed by reversed phase HPLC using a 100 min gradient.

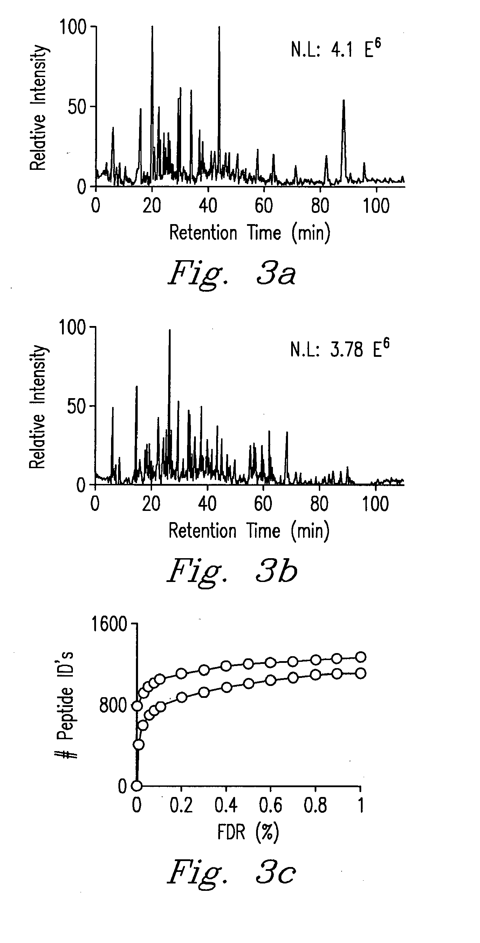

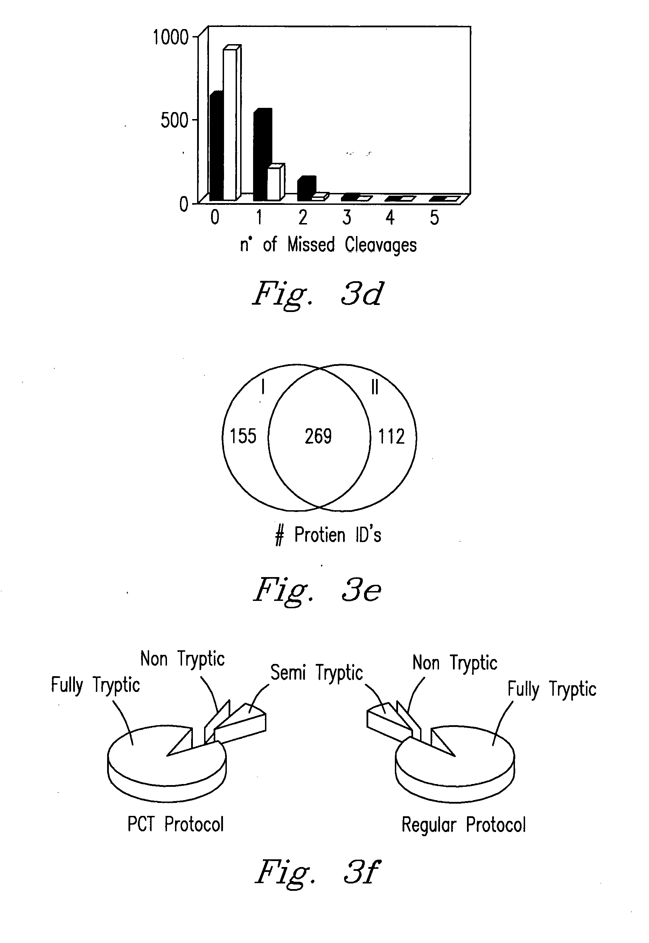

The total ion current chromatograms from the LC-MS/MS analyses of the trypsin digestion using the traditional method at typical ambient pressure and the PCT assisted digestion are provided in Figure 3 (a and b) for comparison. Note that the chromatograms display very similar intensity profiles in spite of different digestion reactions; however, the total number of identified peptides obtained using the pressure protocol is slightly higher (-10%) than that obtained by the conventional method (Figure 3c). The results from a more constrained study of the identified peptides, i.e., false discover rate (FDR) <1% showed that the number of peptides with more than one missed cleavage was much lower for the traditional protocol (Figure 3d), while approximately more than 95% of the PCT-assisted digested peptides had less than two missed cleavages.

Nevertheless, both experiments had a wide overlap in terms of identified proteins, with the PCT-assisted digestion protocol producing more protein identifications (Figure 3e). The number of non-tryptic peptides was insignificant in both samples (Figure 3f) and within the error limits we set for identifying peptides (i.e., <1% FDR). The population of semi-tryptic peptides, defined as a peptide with one end that is not tryptic, is very similar for both digestions.

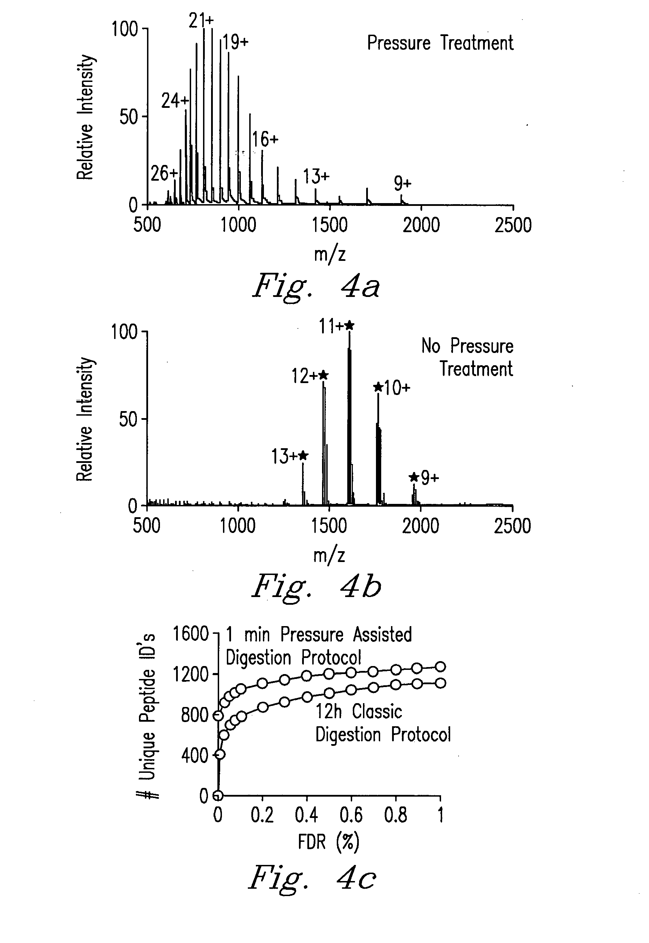

Finally, to test the hypothesis that a better digestion yield is due to the unfolding effect caused by pressure, myoglobin was dissolved in 12 mM ammonium bicarbonate and subjected to 10 kpsi for 60 s prior to direct infusion into the mass spectrometer. For comparative purposes, a native myoglobin protein sample not subjected to pressure treatment was also analyzed by TOF-MS. As shown in Figure 4, the spectra for pressure-treated and non-pressure-treated proteins taken at a neutral pH are completely different. Remarkably, the charge states corresponding to the native protein are much lower than those corresponding to the pressure treated protein. The native protein yielded the maximum signal at a MW of 17,568 Da, whereas the distribution of the charge states for the pressure-treated protein shifted to higher charge states, and the MW corresponded to 16,952 Da. This finding indicates a loss of the heme group (-616 Da) and complete denaturation of the protein due only to the pressure treatment; unfolding of the native protein permitted a reduction of charge-charge repulsion forces, which in turn allowed a larger degree of protonation.

These results demonstrate how an increase in pressure can dramatically increase the rate of the enzymatic digestion of proteins in proteomic samples. Among the advantages afforded by the present invention include, automated sample preparation, high sample throughput (up to three samples per minute in our setup) without compromising the digestion yields, high reproducibility, no aerosolization (a common effect that occurs when HIFU is applied), and the acquisition of results comparable to those obtained using regular digestion protocols but in a much shorter time-frame (ie, 1 min). Since digestions can be completed at 20 °C, undesired protein modification can be avoided.

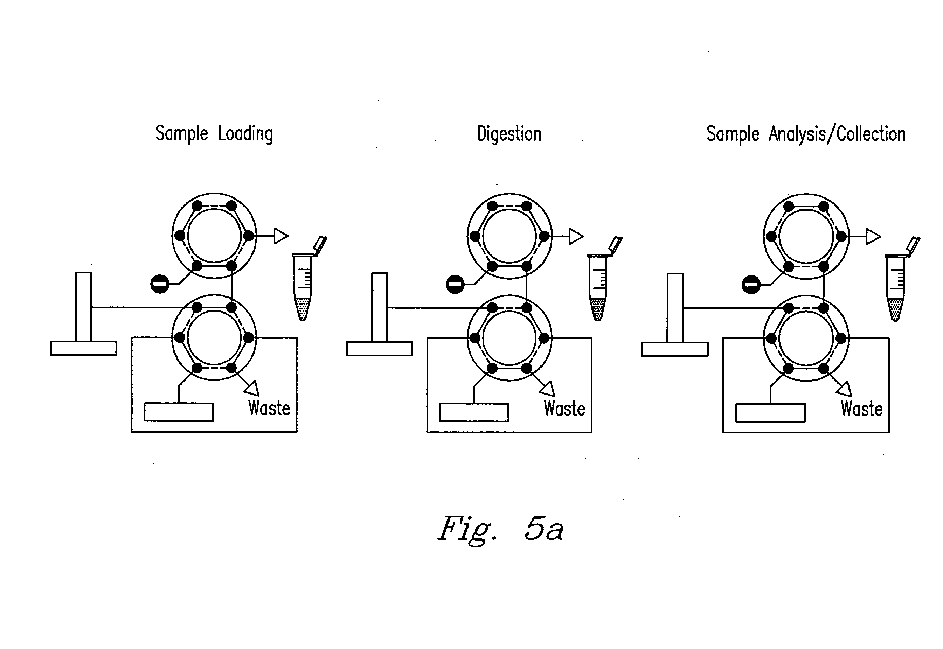

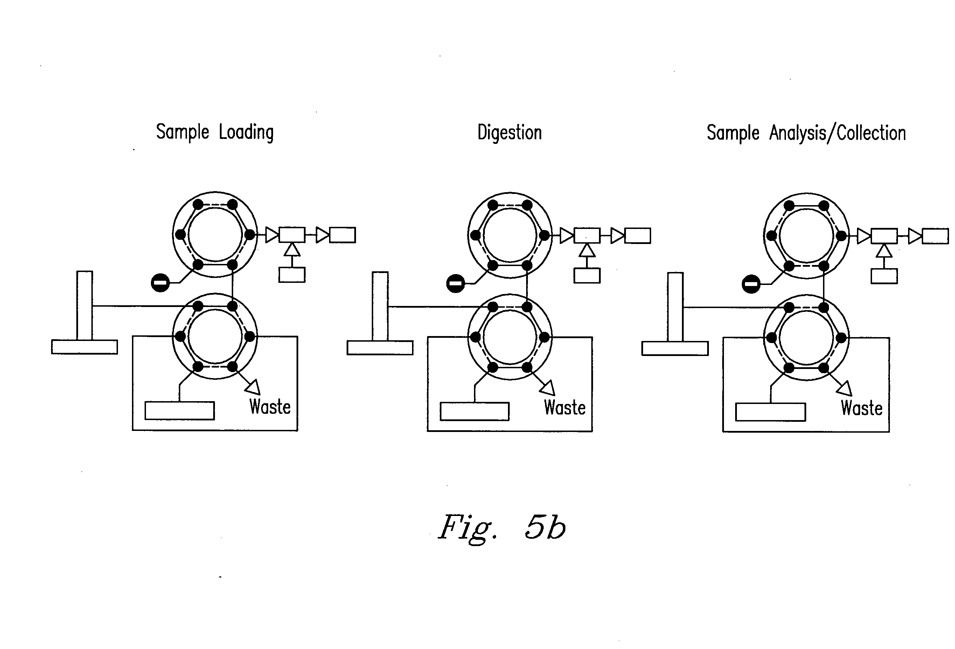

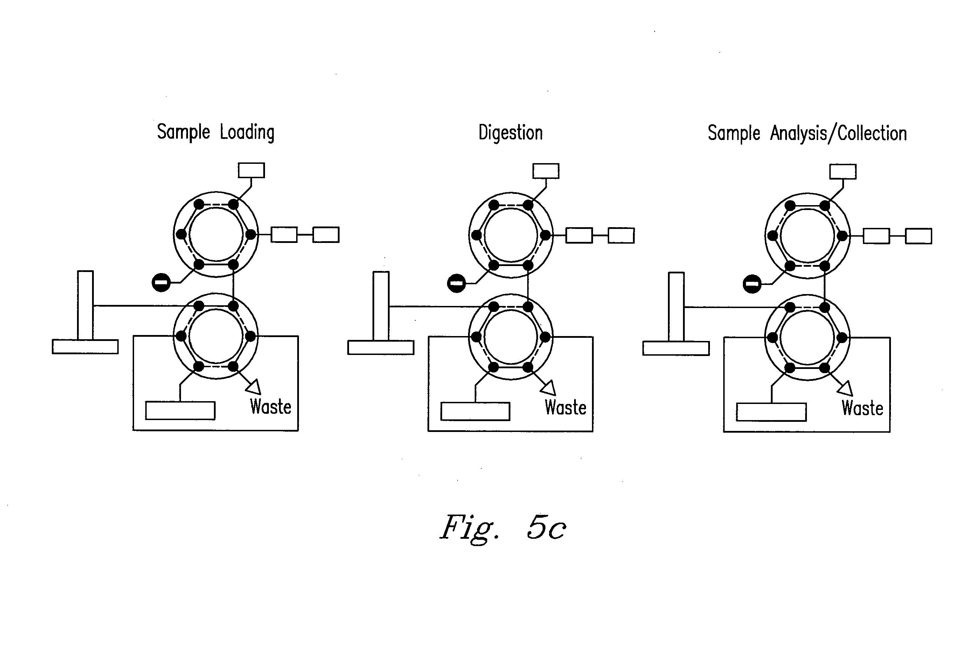

The methods of present invention can be implemented through systems such as the systems shown in Fig. 5a-5c. Figure 5a shows an off-line system, while Figures 5b and 5c show on-line digestion systems that reduce the number of sample manipulation steps for high throughout proteomics. A pressurized sample loop is included in a liquid chromatography-based separation system wherein both sample and enzyme (e.g., trypsin) can be simultaneously introduced to produce a complete, an ultra-fast digestion. The fluidic components of the system consist of a 6-port injection valve with a 5 µL sample loop, and a 4-port valve that are rated to 15,000 psi. A 10,000 psi syringe pump was used to supply mobile phase to the system. The fast on-line digestion system (FOLDS) was operated at a constant pressure of 7,000 psi and used water as a mobile phase. Several modifications where implemented to couple the FOLDS on-line to a mass spectrometer. In the apparatus shown in Fig 5b, a second syringe pump filled with 90% acetonitrile and 1% formic acid was used to re-acidify the sample just prior to ESI. In the third configuration, the FOLDS was coupled to an Agilent LC 1100 system equipped with a nano-flow pump. Peptides were eluted using a gradient from 10 to 60% solvent B (Solvent A: 0.5% formic acid. Solvent B: 0.5% formic, 80% acetonitrile).

The operation of the FOLDS and details showing valve, port, and sample loop placement are shown in Figure 5. System functionality is described in three parts, corresponding to stages of sample processing: loading, digestion, and analysis or collection. Figure 5 illustrates that initially during the sample loading stage the loop is filled with 5 µl of sample and dissolved trypsin. To begin the accelerated protein digestion, the first valve is switched to inject position that enables system pressurization to 7,000 psi, but the liquid flow to the rest of the system is blocked since the second valve is in the load position and the port is closed. In the digestion stage, the sample loop becomes a reaction chamber and digestion is allowed to continue for 1 to 3 minutes. When the pressure-assisted digestion is finished, the second valve is switched to the inject position, initiating the sample analysis stage. The digested sample is either directly infused into a mass spectrometer, collected for off-line analysis, or directed to a reversed phase column for chromatographic separation.

Initially the ODS was employed in an off-line mode (Figure 5a) to characterize digestion efficiency. Once the samples were collected and dried down, they were resuspended in 10 *L of 40% MeOH:Water, 0.1% formic acid buffer and electrosprayed using a TriVersa Nanomate into a modified Bruker 12 T APEX-Q FTICR mass spectrometer, as previously described¹¹. Each mass spectrum was recorded every 2 s, and an average of three mass spectra was used for data analysis. For on-line applications, two different set ups were used. As shown in Figure 5b, the ODS was coupled to a platform incorporating a home-built IMS apparatus with an Agilent 6210 oTOF MS. In another set of experiments, the ODS was coupled to a capillary RPLC separation followed by an ion trap and operated as previously described (Figure 5c).

Proteins were solubilized in a 12.5 mM ammonium bicarbonate buffer (pH 8.2) and mixed with the sequencing grade modified trypsin, in a 1:50 enzyme-to-substrate ratio. The mixture was then loaded into the pressurized system. Digests were either collected for off-line experiments or analyzed directly using MS. Bovine serum albumin was first reduced with 10 mM DTT at 37 ºC during 1 hour and alkylated with 50 mM IAA at room temperature for 45 min. The *Shewanella oneidensis* was prepared as described elsewhere and used as a control. Otherwise, the soluble proteome was prepared in the same way as described above.

For those analyses where only high resolution MS was employed, protein identifications were carried out using a MASCOT search engine. Due to low complexity of the samples, if the score was outside of the uncertain zone the protein was considered identified. For the MS/MS analysis, a SEQUEST™ database search engine was used. For calculation of the error rates associated with peptide identifications, the same method as published before was used.

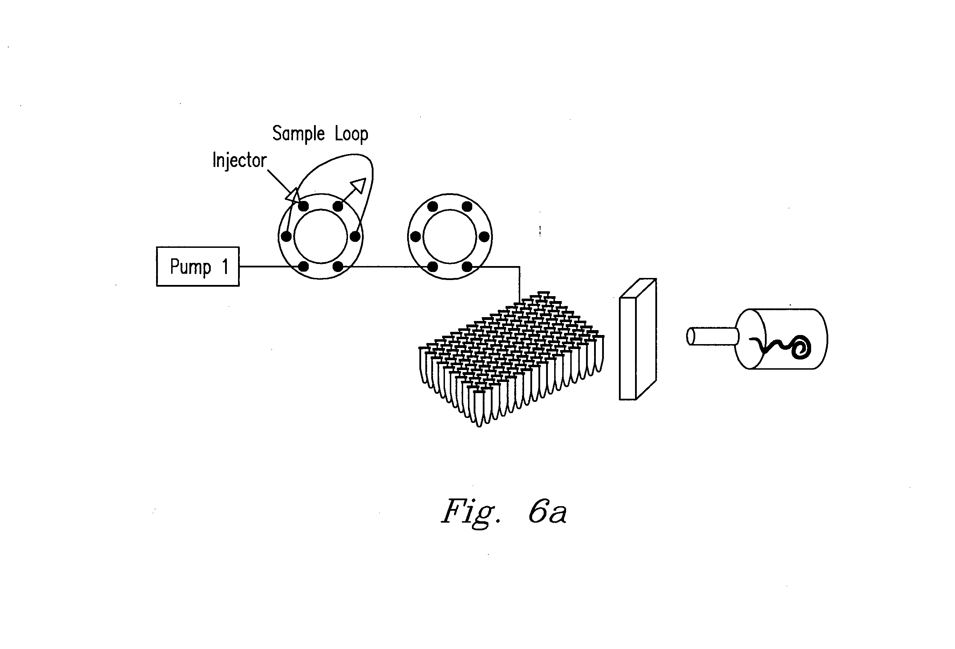

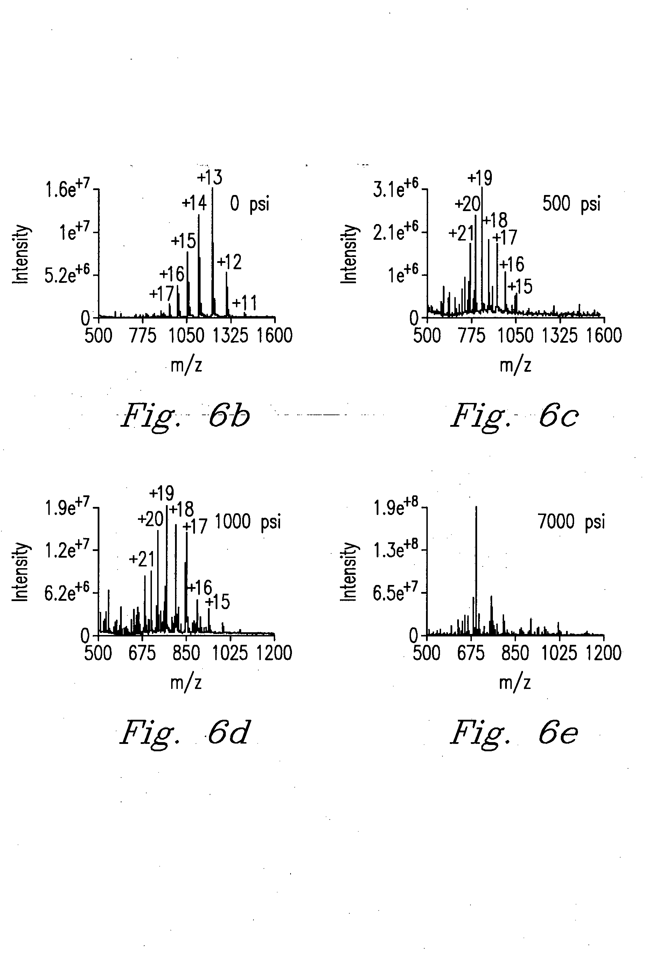

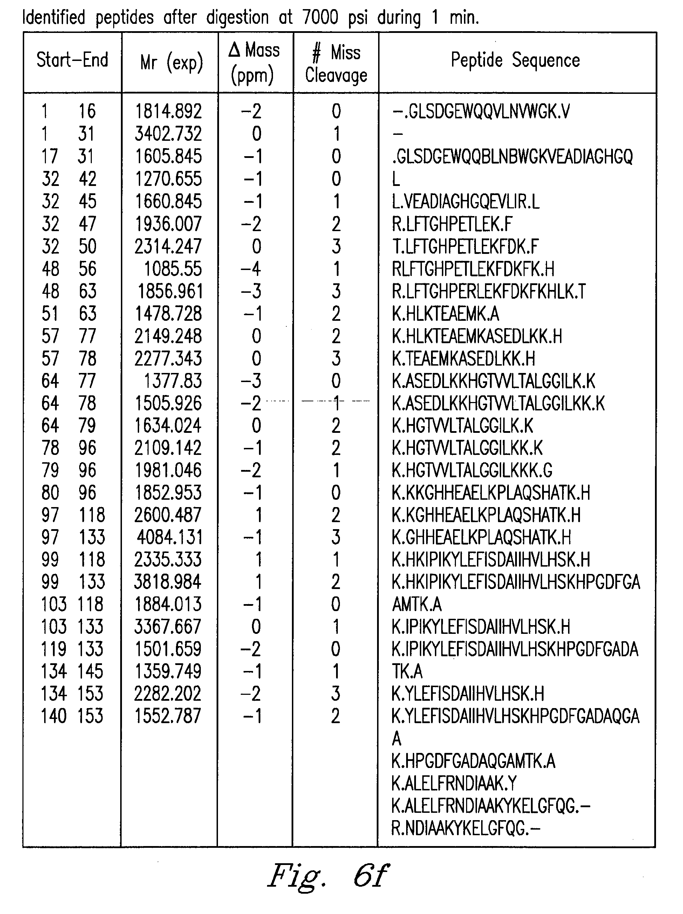

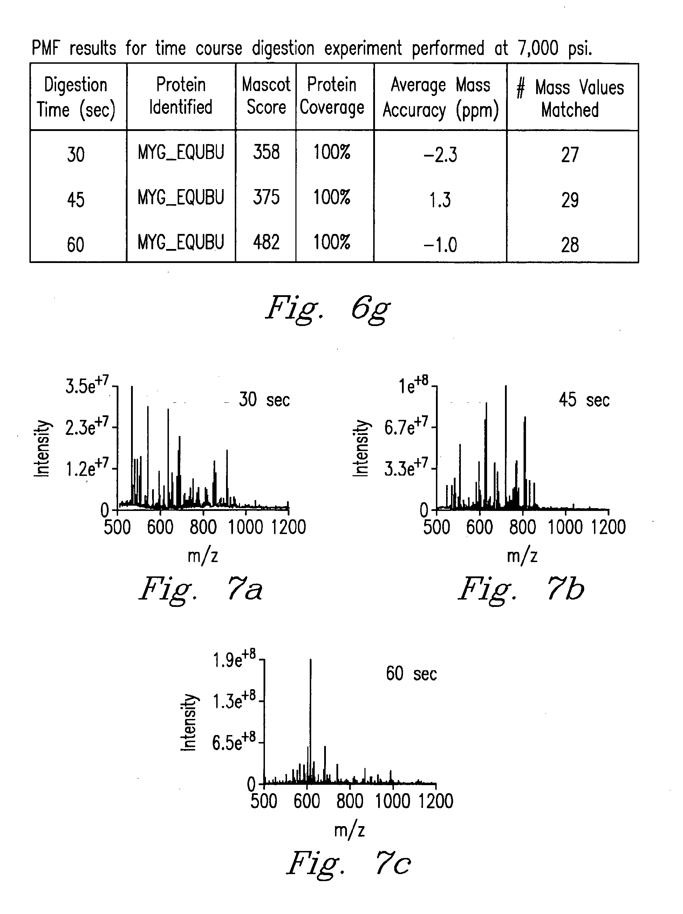

The first experiment used 1 pmol of myoglobin in 12 mM ammonium bicarbonate with trypsin that was injected into the FOLDS (Figure 5a). The pressures were applied during 1 min and varied from 0 to 7000 psi. After a minute, the sample was collected in a reaction tube and analyzed using ESI-chip-assisted direct infusion into the 12T FTICR MS (Figure 6). The use of FOLDS allowed us to simultaneously detect any non-digested intact proteins along with proteolytic peptides. When no pressure was applied, the protein eluted from the FOLDS was detected with charge states corresponding to 11 to 15. When pressure in the FOLDS was increased to 500 psi, higher charge states for the protein, but no peptide fragments, were observed. This is most likely due to a dramatic change in the protein tertiary structure resulting in previously unexposed sites being protonated. In the third experiment, pressure was increased to 1000 psi. The MS analysis revealed that digestion and denaturation processes began to occur, showing a mixture of both peptides and the intact protein. The peptides produced and identified at this pressure provide 100% protein coverage with less than three missed cleavages. Finally, in order to confirm a correlation between higher pressures and a digestion rate, we increased the pressure in the FOLDS up to 7000 psi. As a result, complete protein digestion was achieved in one minute, as shown in Figures 6b-6f.

Since 7000 psi pressure facilitated complete and rapid digestion, we further explored digestion kinetics. Figure 7a-7c shows that even at 30 sec reaction time a satisfactory digestion was achievable. By analyzing peptides generated in 30 sec digestion with the high mass accuracy 12 T FTICR MS, we were able to obtain good protein coverage but with a considerable level of missed cleavages.

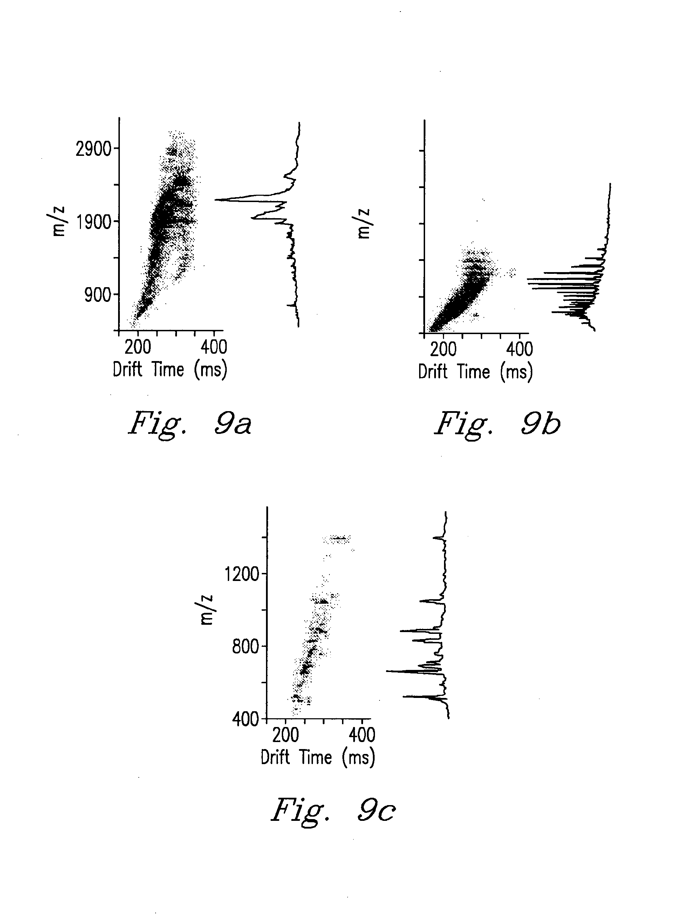

To increase the sensitivity of the device, an organic buffer (90% MeOH, 1% Formic acid) delivered by an independent syringe pump was mixed with the FOLDS eluent at the ESI emitter yielding an improved ionization efficiency. Back flow of the acidified solvent was prevented by the higher pressure of the FOLDS pump. A schematic view of Myoglobin digestion was investigated, using one minute pressure application with and without trypsin. Figure 9 shows that the myoglobin digestion results obtained with the IMS-TOF-MS were consistent with those reported for FTMS. The total analysis time from the start of injection until spectrum acquisition was less than 90 sec. The key contributor to this speed gain is the ability of the IMS-TOF MS to separate ions quickly in the gas phase based mainly on their charge and gas-kinetic cross section.

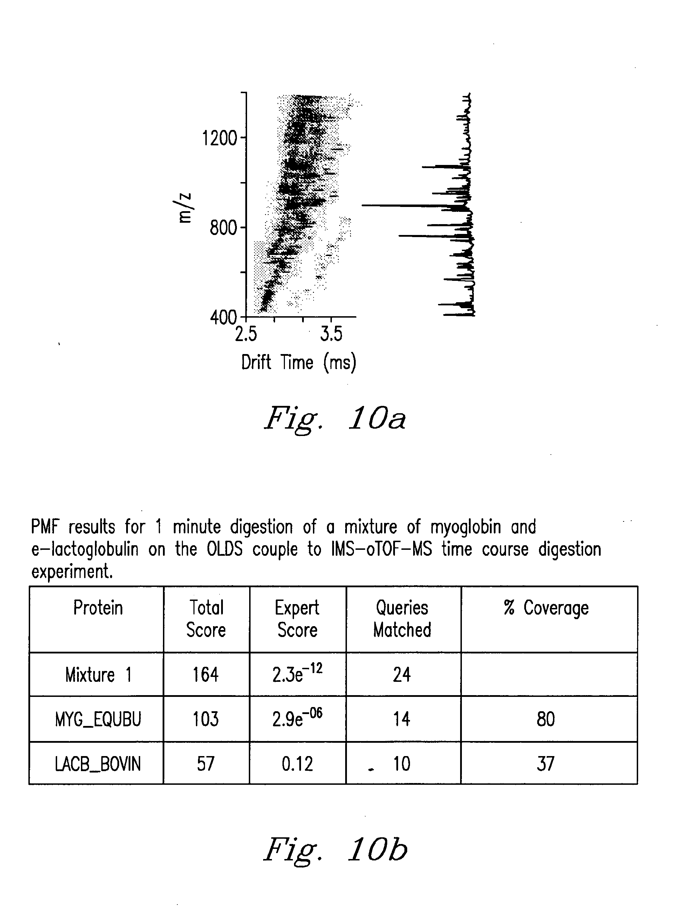

Figure 10 shows the result of analysis of an equimolar mixture of myoglobin and β-lactoglobulin (1 pmol each) using the FOLDS coupled to the IMS-TOF MS. The mixture was digested under the elevated pressure for one minute, and the products were delivered to the IMS-TOF MS as described above. The peptide mass fingerprinting analysis yielded identifications of both proteins with high MASCOT scores as well as high protein coverage, as summarized in the table found in Fig. 10b. One of the disadvantages of the ODS in the on-line direct infusion configuration is that proteomic sample processing often requires the use of salts and chaotropes, which can generate increased chemical noise and reduce ionization efficiency that impairs accurate protein identifications.

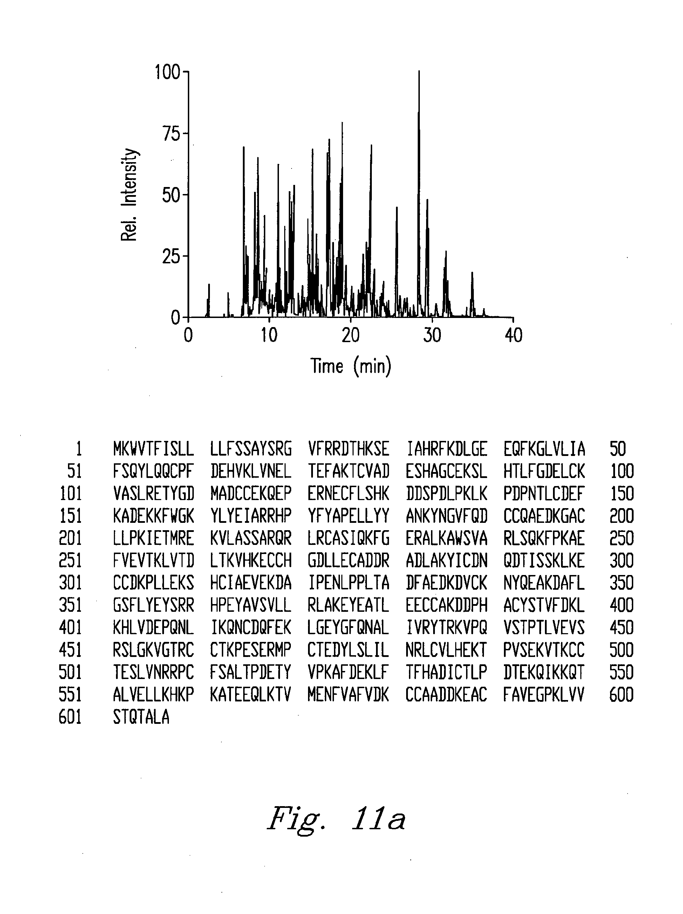

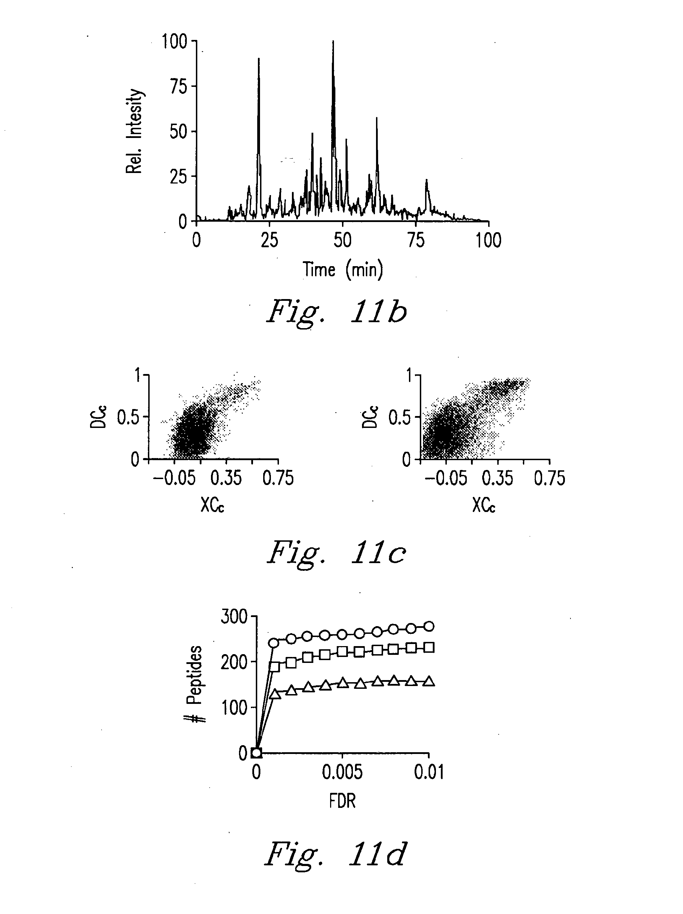

To address this limitation and achieve higher sensitivity, we coupled the FOLDS to RPLC separation to desalt the post-digestion sample. In this study, 10 pmol of BSA was reduced and alkylated in 8M urea, diluted 10-fold with 12.5 mM ammonium bicarbonate, and mixed with trypsin. 1 pmol of the resulting solution was injected into the FOLDS. After a 1 minute digestion at 7,000 psi, the peptide products were loaded onto a reverse-phase column at a flow rate of ca.10 µL/min and then separated using a 20 min gradient under the conditions described in the Experimental section. The LC effluent was analyzed with an LCQ mass spectrometer in MS/MS mode. Figure 11a shows that high protein coverage, ca. 70% of the amino acid sequence, was achieved and the 48 identified peptides were all homogenously distributed across the protein sequence.

In one experiment, a bacterial proteome of *Shewanella oneidensis* was studied. The soluble proteome was resuspended in 25 mM ammonium bicarbonate, trypsin was added in a 1:50 enzyme:protein ratio and 5 µg of total protein amount was injected into the system and digested at 7,000 psi for 1 minute. Proteolytic digests obtained using the conventional protocol and the FOLDS were analyzed for comparison with and without reduction/alkylation procedures. In the conventional proteome digestion with trypsin, a 5 µg aliquot of *Shewanella oneidensis* was subjected to regular digestion for 5 hours at ambient pressure with no chaotropes added and without reduction or alkylation of the proteins. The same sample was then digested with the FOLDS without chaotropes and reduction/alkylation. As a result, the number of identified peptides with the conventional procedure was found to be lower than that identified from the FOLDS-processed sample.

Since the previous myoglobin studies had shown that pressure denatures proteins, concurrently accelerating reaction kinetics, a second set of experiments was aimed at evaluating the effect of denaturation. A 5 µg of the *Shewanella onidensis* proteome was reduced and alkylated in the presence of 8 M urea and then subjected to trypsin digestion using both the conventional and FOLDS protocols. Figure 11b shows that the number of identified peptides with the conventional procedure was close to those obtained with the FOLDS. This study indicates that not only the increased pressure accelerates digestion rates also because acts as a denaturing agent without the need of adding chaotropes, facilitating the formation of the complex between the enzyme molecules and the substrate. Furthermore, these data and the structural changes observed for myoglobin treated at high pressure suggest that not only does increased pressure accelerate proteolytic digestion, but it also denatures the protein like a chaotrope. Further research is necessary to confirm this hypothesis. Nevertheless since pressure denatures proteins, but trypsin is still working, we believe that sequencing trypsin is engineered on the way that its catalytic triad is tremendously stable to high temperature and in the same way to pressure.

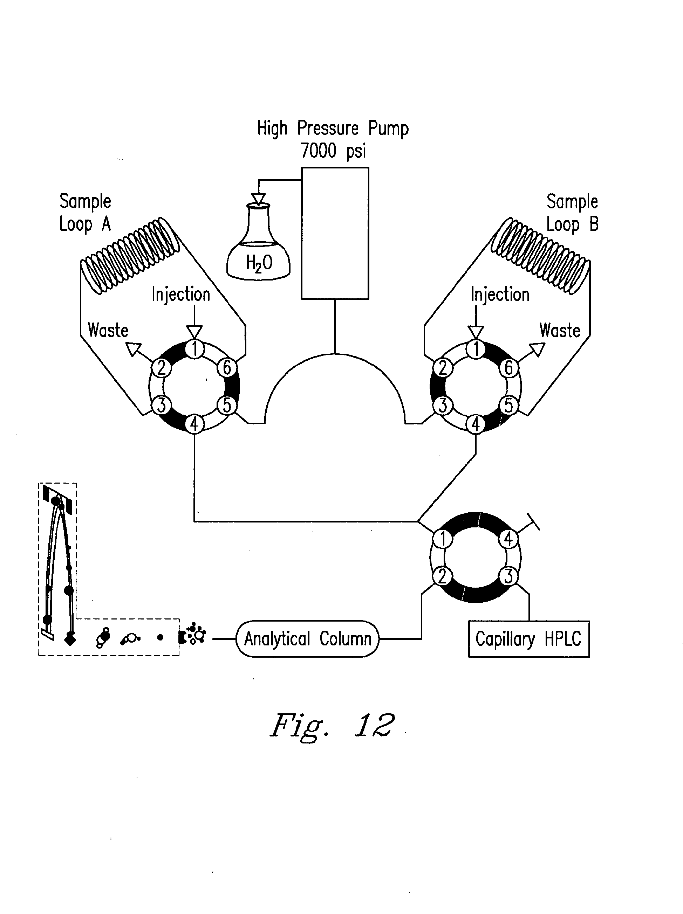

In another set of experiments, we demonstrate the possibility of isotopically labeled peptides using a dual on-line digestion system by changing regular water for ¹⁸O enriched water in the digestion buffer Figure 12. Which is a enzymatically catalyzed reaction where all generated peptides can be labeled with at least one ¹⁸O atom to a maximum of two in the carboxy terminus. The reaction is described in the scheme above where a protease incorporates two O-18 atoms by reversible binding of peptides by enzyme molecules of the serine protease family.

Introducing stable isotopes allows global quantitative comparisons in between different samples due to the mass differences that isotopes introduce in each sample. As proof of concept, Figure 12 shows an equimolar mixture of BSA peptides. Two equal aliquots of BSA protein have been on-line digested, isotopically labeled and analyzed using LC-MS. The results obtained with the optimized workflow incorporating FOLDS-enhanced digestion and labeling are comparable to those obtained using traditional, more time consuming proteomics workflows, indicating that data quality is not compromised by going faster. This dual FOLDS system may provide significant improvement in the overall protein analysis throughput for biological applications involving large numbers of samples, such as for clinical studies of biomarker discovery.

The use of FOLDS in conjunction with MS for the identification of digestion products has accomplished several objectives. First, extended incubation times are no longer needed for effective protein digestion since the application of high pressure accelerates the proteolysis kinetics. Second, the use of trypsin in solution eliminates non-specific binding observed with immobilized enzymes. Third, the coupling of the FOLDS to IMS-TOF MS yields an analysis platform with the capacity to rapidly detect large numbers of peptide ions in an extremely fashion way very useful in single protein characterization or monitoring. This peak capacity can be further increased by coupling capillary RPLC separation to the IMS-TOF MS instrument. In all of the configurations reported, many sample handling steps are eliminated, making the automation of these methods feasible.

## Claims

1. A method for selectively accelerating protein fragmentation **characterized by** co-applying pressure in more than one pressure cycle between low pressure and a high pressure and at least one trypsin to a protein to obtain a processed sample in a preselected period of time.

2. The method of claim 1 wherein the pressure cycle points are in the range of 0.034 to 6895 bar (0.5 psi to 100 kpsi).

3. The method of claim 1 wherein the high pressure is in the range of 344 to 2413 bar (5 to 35 kpsi).

4. The method of claim 1 wherein said preselected period of time is between 5 seconds and 1800 seconds.

5. The method of claim 1 wherein said protein is present in a solid support.

6. The method of claim 5 wherein said protein is present in a gel matrix.

7. The method of any of the aforementioned claims further comprising the step of: treating said protein with isotopes in addition to said pressure and trypsin, to create a preselected mark on said processing sample.

## Patentansprüche

1. Ein Verfahren zur selektiven Beschleunigung der Protein-Fragmentierung, **dadurch gekennzeichnet, dass** Druck in mehr als einem Druckzyklus, zwischen geringem Druck und hohem Druck, und wenigstens ein Trypsin zusammen an einem Protein angewendet werden, um eine verarbeitete Probe in einer vorgewählten Zeitspanne zu erhalten.

2. Das Verfahren nach Anspruch 1, worin die Punkte des Druckzyklus in einem Bereich von 0,034 bis 6895 bar (0,5 psi bis 100 kpsi) liegen.

3. Das Verfahren nach Anspruch 1, worin der hohe Druck in einem Bereich von 344 bis 2413 bar (5 psi bis 35 kpsi) liegt.

4. Das Verfahren nach Anspruch 1, worin die vorgewählte Zeitspanne zwischen 5 Sekunden und 1800 Sekunden liegt.

5. Das Verfahren nach Anspruch 1, worin das Protein in einem festen Träger vorliegt.

6. Das Verfahren nach Anspruch 5, worin das Protein in einer Gelmatrix vorliegt.

7. Das Verfahren nach einem der vorgenannten Ansprüche, ferner die Schritte umfassend: Behandeln des Proteins mit Isotopen zusätzlich zu Druck und Trypsin, um eine vorgewählte Markierung auf der verarbeiteten Probe zu erstellen.

## Revendications

1. Procédé pour sélectivement accélérer la fragmentation d'une protéine, **caractérisé par** la co-application d'une pression dans plus d'un seul cycle de pression entre une basse pression et une haute pression et d'au moins une trypsine à une protéine afin d'obtenir un échantillon traité dans une période présélectionnée.

2. Procédé selon la revendication 1, dans lequel les points du cycle de pression sont dans la plage de 0,034 à 6 895 bars (0,5 psi à 100 kpsi).

3. Procédé selon la revendication 1, dans lequel la haute pression est dans la plage de 344 à 2 413 bars (5 à 35 kpsi).

4. Procédé selon la revendication 1, dans lequel ladite période présélectionnée est comprise entre 5 secondes et 1 800 secondes.

5. Procédé selon la revendication 1, dans lequel ladite protéine est présente dans un support solide.

6. Procédé selon la revendication 5, dans lequel ladite protéine est présente dans une matrice de gel.

7. Procédé selon l'une quelconque des revendications précédentes, comprenant en outre l'étape de : traitement de ladite protéine avec des isotopes, en plus de ladite pression et trypsine, afin de créer un marqueur présélectionné sur ledit échantillon traité.
